# EUROPÄISCHE PATENTSCHRIFT

(11) **EP 1 506 153 B1**
(45) Veröffentlichungstag und Bekanntmachung des Hinweises auf die Patenterteilung: **22.02.2006**
(21) Anmeldenummer: 03749807.8
(22) Anmeldetag: 02.05.2003
(51) Int. Cl.: C07C 51/44

(54) **VERFAHREN ZUR HERSTELLUNG EINER WÄSSRIGEN ALKALIACRYLAT-LÖSUNG**
METHOD FOR PRODUCING AN AQUEOUS ALKALI ACRYLATE SOLUTION
PROCEDE DE PRODUCTION D'UNE SOLUTION AQUEUSE D'ACRYLATE ALCALIN

(30) Priorität: 07.05.2002 DE 10220494
(43) Veröffentlichungstag der Anmeldung: 16.02.2005
(73) Patentinhaber: BASF Aktiengesellschaft, 67056 Ludwigshafen (DE)
(72) Erfinder: FUCHS, Eberhard, 67227 Frankenthal (DE); MARTAN, Hans, 67227 Frankenthal (DE); MÜLLER-ENGEL, Klaus, Joachim, 76297 Stutensee (DE)
(86) Internationale Anmeldenummer: PCT/EP2003/004602
(87) Internationale Veröffentlichungsnummer: WO 2003/095410

(56) Entgegenhaltungen:
- DE-A- 3 432 082
- US-A- 4 219 389
- DATABASE CA [Online] CHEMICAL ABSTRACTS SERVICE, COLUMBUS, OHIO, US; YADA, AKIRA ET AL: "Aqueous acrylic or methacrylic acid salt solutions" retrieved from STN Database accession no. 84:136272 XP002252787 & JP 50 142511 A (TOA GOSEI CHEMICAL INDUSTRY CO., LTD., JAPAN) 17. November 1975 (1975-11-17)

## Beschreibung

Die vorliegende Erfindung betrifft ein Verfahren zur Herstellung einer wässrigen Alkaliacrylat-Lösung, bei dem man aus einem Acrylsäure enthaltenden Gemisch Acrylsäure destillativ abtrennt und aus der destillativ abgetrennten Acrylsäure und einer wässrigen Lösung eines basischen Alkalisalzes eine wässrige Alkaliacrylat-Lösung erzeugt.

Unter dem Begriff Alkaliacrylat sollen in dieser Schrift die Alkalisalze von Acrylsäure verstanden werden.

Unter einer wässrigen Alkaliacrylat-Lösung soll eine wässrige Lösung verstanden werden, die wie eine solche wässrige Lösung beschaffen ist, die wenigsten ein Alkaliacrylat gelöst enthält.

Acrylsäure wird im allgemeinen durch heterogen katalysierte Gasphasenoxidation von Propen oder Propan mit molekularem Sauerstoff erzeugt (vgl. z.B. Ullmann's Enzyclopedia of Ind. Chem. 5^{th} ed. on CD-ROM, "Acrylic acid and derivatives, 1.3.1 Propenoxidation", Wiley-VCH Weinheim, 1997; K. Weisärmel, H.-J. Arpe "Industrielle Org. Chem.", 4. Auflage, VCH Verlagsgesellschaft, Weinheim 1994, S. 315-17 sowie DE-A 2943707, DE-C 1205502, EP-A 117146, EP-A 293224, GB-A 1450986, DE-A 10131297 und DE-A 10122027).

Das im Rahmen der heterogen katalysierten Gasphasenoxidation entstehende Produktgasgemisch enthält neben der Hauptkomponente Acrylsäure Nebenkomponenten wie z.B. Essigsäure und Propionsäure, eine Reihe von Aldehyden wie beispielsweise Furfurale, Benzaldehyd, Formaldehyd, Acrolein, Acetaldehyd und Propionaldehyd, weiterhin Protoanemonin sowie diverse ungesättigte oder aromatische Carbonsäuren und deren Anhydride wie z. B. Benzoesäure, Maleinsäure, Maleinsäureanhydrid und Phthalsäureanhydrid.

Die Mehrzahl dieser Nebenkomponenten erweist sich bei einer Folgeverwendung der Acrylsäure als nachteilig.

Dies gilt insbesondere dann, wenn man beabsichtigt, die Acrylsäure für die Herstellung von wasserabsorbierenden Harzen zu verwenden.

Die Herstellung von wasserabsorbierenden Harzen auf der Basis von Acrylsäure erfolgt bekanntermaßen vorwiegend durch radikalische Polymerisation wässriger Monomerlösungen, die im wesentlichen Acrylsäure als radikalisch polymerisierbares Monomeres enthalten, wobei in der Regel wenigstens eine Teilmenge der Acrylsäure in der wässrigen Monomerlösung wie gelöstes Alkaliacrylat vorliegt. Monomere sollen hier ganz allgemein chemische Verbindungen sein, die wenigstens eine ethylenisch ungesättigte Doppelbindung aufweisen. Die Polymerisation selbst kann dabei z.B. als Lösungs- oder als Gel-Polymerisation in homogener wässriger Phase oder auch als Suspensionspolymerisation ausgeführt werden, wobei die wässrige Monomerlösung die disperse Phase bildet. Die so erhältlichen Hydrogele werden nachfolgend in der Regel noch oberflächenvernetzt. In getrockneter Form bilden sie dann Pulver, die eine ausgeprägte Fähigkeit zur Absorption von Wasser aufweisen und z.B. in Windeln oder Hygieneartikeln Verwendung finden. Man spricht deshalb in diesem Zusammenhang auch von Superabsorbern.

Die vorgenannten Nebenkomponenten können nun nicht nur bereits den Polymerisationsvorgang als solchen negativ beeinflussen (sie können z.B. die Polymerisationsgeschwindigkeit oder die Höhe des polymeren Molekulargewichtes negativ beeinflussen), sondern in aller Regel ist auch ihre Anwesenheit im gebrauchsfertigen Superabsorber unerwünscht.

Ausgehend vom Produktgasgemisch der heterogen katalysierten Gasphasenoxidation erfolgt die Herstellung einer wässrigen Alkaliacrylat-Lösung, die sich zur Herstellung von Superabsorbern eignet, daher üblicherweise so, dass man die im Produktgasgemisch enthaltenen Nebenkomponenten wenigstens teilweise von der Acrylsäure abtrennt.

Dazu sind zahlreiche Verfahren aus dem Stand der Technik bekannt. Beispielsweise kann man eine Grundabtrennung der Acrylsäure aus dem Produktgasgemisch dadurch vornehmen, dass man die Acrylsäure in ein geeignetes Absorptionsmittel (z.B. Wasser oder ein Gemisch aus 70 bis 75 Gew.-% Diphenylether und 25 bis 30 Gew.-% Diphenyl) unter Erhalt eines Acrylsäure enthaltenden Absorbats absorptiv aufnimmt (vgl. z.B. EP-A 297445 und DE-PS 2136396). Durch nachfolgende, überwiegend destillative Trennverfahren, läßt sich anschließend aus dem Absorbat eine Acrylsäure abtrennen, die eine erhöhte Reinheit aufweist. Alternativ kann das Produktgasgemisch der Gasphasenoxidation auch fraktioniert kondensiert werden, wie es z.B. die DE-A 19740253 beschreibt. Die dabei entnommene, bereits vergleichsweise reine, Acrylsäure kann, gegebenenfalls nach einer kristallisativen Zwischenreinigung, bedarfsgerecht destillativ weitergereinigt werden.

Die so erhältlichen Acrylsäuren mit erhöhter Reinheit werden üblicherweise mit einem Lagerpolymerisationsinhibitor (z.B. Hydrochinonmonomethylether) versetzt, der den Zweck verfolgt, in der Acrylsäure gelöst befindlich, eine unkontrollierte, unerwünschte, vorzeitige radikalische Polymerisations zu unterdrücken.

So zubereitet, wird die Acrylsäure in Lagertanks aufbewahrt und nach Bedarf zur Herstellung von Superabsorbern verwendet. Dazu wird aus der polymerisationsinhibierten Acrylsäure und einer wässrigen Lösung eines basischen Alkalisalzes und gegebenenfalls sonstigen Komponenten eine polymerisationsfähige wässrige Alkaliacrylat-Lösung erzeugt (vgl. z.B. JP-A 50142511). Durch Zusatz von Polymerisationsinitiator (z.B. Peroxidverbindungen) sowie gegebenenfalls unter der Einwirkung erhöhter Temperaturen wird dann die radikalische Polymerisation üblicherweise ausgelöst, um das superabsorbierend wirkende Alkalipolyacrylat zu erzeugen.

Nachteilig an der vorstehend beschriebenen Verfahrensweise ist, dass der verwendete Polymerisationsinitiator und der in der Acrylsäure enthaltene Lagerpolymerisationsinhibitor Antagonisten bilden. Ferner ist von Nachteil, dass sich während der Lagerung der Acrylsäure in selbiger durch Michaeladdition an sich selbst Acrylsäureoligomere bilden, unter denen die Diacrylsäure aus gründen der Statistik die größte Bedeutung aufweist. Diese Acrylsäureoligomere sind insbesondere insofern von Nachteil, als sie unter der Einwirkung erhöhter Temperatur monomere Acrylsäure abspalten. D.h., werden sie copolymerisiert, kann dies dazu führen, dass das resultierende Polymerisat nach Beendigung der Polymerisation und Beseitigung von verbliebenen, nicht polymerisierten Monomeren, in unerwünschter, toxikologisch nicht völlig unbedenklicher Weise, wieder monomere Restacrylsäure aufweist. Häufig wird die gelagerte Acrylsäure deshalb vor ihrer Verwendung nochmals einer destillativen Reinigung unterworfen.

Aufgabe der vorliegenden Erfindung war es nun, ein verbessertes Verfahren zur Herstellung einer wässrigen Alkaliacrylat-Lösung, bei dem man aus einem Acrylsäure enthaltenden Gemisch Acrylsäure destillativ abtrennt und anschließend aus der destillativ abgetrennten Acrylsäure und einer wässrigen Lösung eines basischen Alkalisalzes eine wässrige Alkaliacrylat-Lösung erzeugt, zur Verfügung zu stellen, das die vorgenannten Nachteile entweder überhaupt nicht mehr oder nur noch in verminderter Weise aufweist.

Demgemäß wurde ein Verfahren zur Herstellung einer wässrigen Alkaliacrylat-Lösung gefunden, bei dem man aus einem Acrylsäure enthaltenden Gemisch Acrylsäure destillativ abtrennt und aus der destillativ abgetrennten Acrylsäure und einer wässrigen Lösung eines basischen Alkalisalzes eine wässrige Alkaliacrylat-Lösung erzeugt, das dadurch gekennzeichnet ist, dass man
a) das die Acrylsäure enthaltende Gemisch einer Destillationsvorrichtung zuführt,
b) die Abtrennung der Acrylsäure vom Acrylsäure enthaltenden Gemisch in der Destillationsvorrichtung oberhalb der Zuführstelle durchführt und
c) die Erzeugung der wässrigen Alkaliacrylat-Lösung so vornimmt, dass die in der Destillationsvorrichtung abgetrennte Acrylsäure unmittelbar aus der Gasphase in eine wässrige Lösung eines Alkalihydroxids, eines Alkalicarbonats und/oder eines Alkalihydrogencarbonats aufgenommen wird.

Als Alkalihydroxid, -carbonat und/oder -hydrogencarbonat kommen für das erfindungsgemäße Verfahren insbesondere die entsprechenden Salze des Natriums, des Kalium oder eines Gemisches dieser beiden Metalle in Betracht.

Innerhalb der Gruppe der vorgenannten Salze sind die Natriumsalze gegenüber den Kaliumsalzen erfindungsgemäß bevorzugt. Ferner sind innerhalb der Gruppe der vorgenannten Salze die Hydroxide erfindungsgemäß bevorzugt. D.h., erfindungsgemäß besonders bevorzugt wird eine wässrige Natriumhydroxidlösung verwendet.

Der Term "destillative Abtrennung" soll erfindungsgemäß weitestgehend verstanden und sowohl eine einfache Destillation, d.h. eine Destillation, bei der im wesentlichen keine kondensierte Phase im Gegenstrom zum aufsteigendem Dampf geführt wird, als auch eine Rektifikation, bei der kondensierte Phase im Gegenstrom zum aufsteigendem Dampf geführt wird und mit diesem in ausgeprägtem Stoffaustausch steht, umfassen.

In einfacher Weise läßt sich die erfindungsgemäße Abtrennung der Acrylsäure z.B. so realisieren, dass man die Abtrennung oberhalb eines in der Destillationsvorrichtung angebrachten Kaminbodens vornimmt. Dieser ist so gestaltet, dass in ihm durch den Kamin zwar Gasphasen aufsteigen aber keine Flüssigphase von ihm in die Destillationsvorrichtung rücklaufen kann.

Im Raum oberhalb der Kaminöffnung, die in der Regel bedacht sein wird, wird die erfindungsgemäß zu verwendende wässrige Lösung eines Alkalihydroxids, eines Alkalicarbonats und/oder eines Alkalihydrogencarbonats zweckmäßigerweise als feinteilige Tröpfchen versprüht (zerstäubt, zerteilt).

Bei Kontakt derselben mit dem aufsteigenden Acrylsäuredampf (wie er z.B. aus der US-A 4219389 bekannt ist) wird die Acrylsäure in die feinteiligen wässrigen Tröpfchen, die vorzugsweise eine möglichst niedrige Temperatur aufweisen, aufgenommen. Die sich dabei bildende wässrige Alkaliacrylatlösung sammelt sich auf dem Kaminboden und kann von selbigem kontinuierlich entnommen werden. Als Tröpfchengröße kann z.B. eine solche von 0,1 mm bis 5 mm, vorzugsweise 0,3 bis 1 mm angewendet werden. Vorzugsweise weist die erfindungsgemäß zu verwendende zerstäubte wässrige Lösung des basischen Alkalisalzes bei der vorstehend skizzierten Verfahrensweise eine Temperatur im Bereich von 10 bis 60 °C, besonders bevorzugt im Bereich von 20 bis 45 °C auf.

Zur Zerstäubung der wässrigen Alkalisalzlösung können z.B. Zerstäuberdüsen eingesetzt werden, wie sie z.B. die DE-A 19924533 beschreibt.

Derartigen Düsen kann die wässrige Alkalilösung, zweckmäßigerweise bereits vorgekühlt, z.B. unter Druck zugeführt werden. Die Zerteilung der wässrigen Alkalilösung kann dabei dadurch erfolgen, dass sie nach Erreichen einer bestimmten Mindestgeschwindigkeit in der Düsenbohrung entspannt wird. Ferner können für den vorgenannten Zweck Einstoffdüsen wie z.B. Drallkammern (Hohl- oder Vollkegeldüsen) verwendet werden (z.B. von der Fa. Düsen-Schlick GmbH, DE, oder von der Sprayring Systems Deutschland GmbH).

Die Zerstäubung der wässrigen Alkalisalzlösung kann dabei erfindungsgemäß auch so durchgeführt werden, dass durch eine Düse eine konzentrierte wässrige Alkalisalzlösung und durch eine andere Düse eine verdünnte wässrige Alkalisalzlösung oder Wasser geführt wird. Durch gezielte Wahl des Verhältnisses der beiden Sprühmengen läßt sich der gewünschte Neutralisationsgrad nach Belieben einstellen.

Alternativ kann man zum Zerstäuben der erfindungsgemäß zu verwendenden wässrigen Alkalisalzlösung auch Prallzerstäuber verwenden. Bei Prallzerstäubern wird die Zerstäubung dadurch bewirkt, dass wenigstens ein Strom an wässriger Alkalisalzlösung auf wenigstens einen zweiten Strom an wässriger Alkalisalzlösung und/oder Wasser und/oder auf eine Prallplatte trifft.

Erfindungsgemäß bevorzugt sind Prallzerstäuber, bei denen die Zerstäubung dadurch bewirkt wird, dass wenigstens ein Strom an wässriger Alkalisalzlösung auf eine Prallplatte (z.B. aus Stahl) trifft (Prallplattenzerstäuber).

Dabei kann der auf die Prallplatte geführte Strom an wässriger Alkalisalzlösung z.B. eine Strömungsgeschwindigkeit von 20 bis 80 km/h aufweisen.

Die Führung der wässrigen Lösung eines erfindungsgemäß zu verwendenden basischen Alkalisalzes erfolgt dabei in zweckmäßiger Weise in einfachen Rohren (z.B. aus Stahl), die gegen das Ende vorzugsweise verjüngt sind.

Der Abstand zwischen der Austrittsöffnung des Rohres und der Prallplatte beträgt erfindungsgemäß zweckmäßig häufig 5 bis 30 cm, vielfach 10 bis 20 cm. Die Größe und die Form der Prallplatte kann in weiten Grenzen variieren. In der Regel ist die Prallplatte rund und ihr Durchmesser beträgt häufig das 1- bis 20-fache, vielfach das 1- bis 5-fache des Durchmessers der Austrittsöffnung des Rohres.
Im Normalfall ist die Prallplatte eben. Die Prallplatte kann aber auch konkav oder konvex geformt sein.

Selbstverständlich kann man bei einer Anwendung des Prinzips der Prallzerstäubung auch eine konzentrierte wässrige Alkalisalzlösung und eine verdünnte wässrige Alkalisalzlösung bzw. Wasser aufeinanderführen.

Die erfindungsgemäß zu verwendende wässrige Lösung eines basischen Alkalisalzes verfolgt zwei Zwecke. Ihr Kälteinhalt dient dem Zweck der direkten Kühlung der gasförmigen Acrylsäure und der Aufnahme der Neutralisationswärme. Die basische wässrige Lösung des Alkalisalzes per se dient darüber hinaus der Aufnahme der Acrylsäure aus der Gasphase und der Bildung einer wässrigen Alkaliacrylat-Lösung.
Diese unmittelbare Erzeugung einer wässrigen Alkaliacrylat-Lösung kann auch als ein Quenchen von Acrylsäure in einer wässrigen basischen Alkalisalzlösung (Quenchflüssigkeit) bezeichnet werden. In der Regel wird der Salzgehalt der Quenchflüssigkeit 10 bis 60 Gew.-%, häufig 30 bis 50 Gew.-% oder etwa 30 bis 40 Gew.-% betragen.

Erfindungsgemäß vorteilhaft wird die erfindungsgemäß erzeugte wässrige Alkaliacrylat-Lösung nicht vollständig der Destillationsvorrichtung entnommen. Vielmehr ist es zweckmäßig zur Unterstützung der Kühl- und Kondensationswirkung eine Teilmenge zu kühlen (z.B. mittels Plattenwärmetauschern) und gekühlt als weitere Quenchflüssigkeit in die Destillationsvorrichtung rückzuführen. In zweckmäßigerweise beträgt die Rückführungstemperatur erfindungsgemäß 10 bis 60 °C, bevorzugt 15 bis 50 °C und besonders bevorzugt 20 bis 45 °C.

Selbstverständlich kann die erfindungsgemäß erforderliche wässrige Lösung eines basischen Alkalisalzes mit der vorstehend beschriebenen Quenchflüssigkeit vereint und nachfolgend gemeinsam als eine Quenchflüssigkeit versprüht werden. Auch kann mit den beiden Quenchflüssigkeiten eine Prallzerstäubung betrieben werden.

Die Rückführung kann bis zu 90 Gew.-% der gebildeten Menge an wässriger Alkaliacrylat-Lösung betragen.

Erfindungsgemäß bevorzugt wird das erfindungsgemäße Verfahren kontinuierlich ausgeführt. Bevorzugt erfolgt erfindungsgemäß die ummittelbare Aufnahme der in der Destillationsvorrichtung abgetrennten Acrylsäure aus der Gasphase in eine wässrige Lösung eines Alkalihydroxids, eines Alkalicarbonats und/oder eines Alkalihydrogencarbonats unmittelbar in der für die nachfolgende Polymerisation vorgesehenen Vorrichtung (z.B. in einem Polymerisationskessel).

Erfindungsgemäß von Vorteil ist, dass die erfindungsgemäße Erzeugung der wässrigen Alkaliacrylat-Lösung quasi in situ erfolgt.

Dies ist insofern von Vorteil, als es bei einer unmittelbaren Weiterverwendung der wässrigen Alkaliacrylatlösung die Möglichkeit eröffnet, auf einen intermediären Zusatz an Polymerisationsinhibitoren ganz zu verzichten. In diesem Fall setzt man der gebildeten wässrigen Alkaliacrylat-Lösung die erforderliche Menge an radikalischem Polymerisationsinitiator zu und erzeugt durch radikalische Polymerisation in an sich bekannter Weise das gewünschte Superabsorberharz. Der Verzicht auf Polymerisationsinhibitor gestattet dabei die Polymerisationsinitiierung mit einer vergleichsweise geringen Menge an radikalischem Polymerisationsinitiator. Aus dieser Einsatzmengenreduzierung (sowohl des Polymerisationsinitiators als auch das Polymerisationsinhibitors) resultiert eine insgesamt wirtschaftlichere Verfahrensweise. Die Möglichkeit des vollständigen Ausschlusses an Polymerisationsinhibitor gestattet zusätzlich die Herstellung zuvor nicht erreichbarer Superabsorberqualitäten. Auch bedingt die beschriebene Verfahrensweise eine Minimierung des Diacrylsäuregehaltes bis zum Polymerisationsbeginn.

Die erfindungsgemäß zu verwendende Menge an wässriger Lösung eines basischen Alkalisalzes wird häufig so bemessen, daß der Neutralisationsgrad in der resultierenden wässrigen Acrylat-Lösung wenigstens 25 mol-%, häufig wenigstens 30 mol-% und vielfach wenigstens 33 mol-%, bezogen auf die enthaltene molare Menge an Acrylsäure, beträgt. Selbstverständlich kann das zur Neutralisation verwendete Alkalisalz auch im molaren Überschuß, bezogen auf die enthaltene molare Menge an Acrylsäure, eingesetzt werden. Ein solcher Überschuß wird in der Regel einen Wert von 10 mol-% bzw. von 5 mol-%, bezogen auf die enthaltene molare Menge Acrylsäure, nicht überschreiten. Anwendungstechnisch zweckmäßig wird man beim erfindungsgemäßen Verfahren vielfach einen Neutralisierungsgrad der wässrigen Alkaliacrylat-Lösung von 30 bis 70 mol-% einstellen.

Selbstredend muß die erfindungsgemäß erzeugte wässrige Alkaliacrylat-Lösung nicht in notwendiger Weise unmittelbar einer radikalisch initiierten Polymerisation zugeführt werden. Vielmehr besteht auch die Möglichkeit, die erfindungsgemäß erzeugte wässrige Alkliacrylat-Lösung vorab ihrer Weiterverwendung für Polymerisationszwecke zwischenzulagern.

Dabei macht sich unter den Gesichtspunkten "unerwünschte radikalische Polymerisation" sowie "Diacrylsäurebildung" vorteilhaft bemerkbar, dass die erfindungsgemäß erzeugte wässrige Alkaliacrylat-Lösung die Acrylsäuremonomeren in einem verdünnten Zustand aufweist. Ferner ist es günstig, daß solche wässrigen Alkaliacrylat-Lösungen stark abgekühlt werden können, ohne daß eine Feststoffbildung erfolgt (Stichwort: Gefrierpunktserniedrigung; reine Acrylsäure besitzt bei Normaldruck einen Festpunkt von etwa 13 °C) .

Dies ist insbesondere dann vorteilhaft, wenn man der erfindungsgemäß erzeugten wässrigen Alkaliacrylat-Lösung zum Zweck einer Lagerung mit erhöhter Sicherheit radikalischen Lagerungsinhibitor zusetzt (z.B. Amine, Nitroverbindungen, phosphor- oder schwefelhaltige Verbindungen, Hydroxylamine, N-Oxide und Chinone). Beispielsweise kommen alle Polymerisationsinhibitoren in Betracht, die in der DE-A 10053086 aufgeführt werden.

Bewährt hat sich in diesem Zusamanenhang insbesondere die Verwendung des Monomethylethers von Hydrochinon.

Mit zunehmender Verdünnung der wässrigen Alkaliacrylat-Lösung sowie mit zunehmender Verringerung der möglichen Lagerungstemperatur ohne Feststoffabscheidung geht ein verringerter Bedarf an Lagerungsinhibitor für eine sichere Lagerung einher.

Eine Feststoffbildung insbesondere der enthaltenen, gegebenenfalls neutralisierten, Acrylsäure ist deshalb zu vermeiden, weil im Rahmen einer solchen Feststoffabscheidung sowohl eine Aufkonzentrierung an Acrylsäure als auch eine Abreicherung an Polymerisationsinhibitor in Acrylsäure einhergeht. Dies kann insbesondere im Rahmen einer nachfolgenden Aufschmelzung von kristallisativ abgeschiedener Acrylsäure zu explosionsartig verlaufender, unerwünschter spontaner Polymerisation von Acrylsäure führen.

In der Regel ist ein Zusatz von 10 bis 250 Gew.ppm an Lagerpolymerisationsinhibitor, häufig 20 bis 60 Gew.ppm, zu den erfindungsgemäß erhältlichen wässrigen Alkaliacrylat-Lösungen ausreichend. Dies gilt insbesondere dann, wenn als Lagerpolymerisationsinhibitor der Monomethylether des Hydrochinons (MEHQ) eingesetzt wird.

Beabsichtigt man eine Anwendung von Lagerpolymerisationsinhibitoren, setzt man diese in zweckmäßiger Weise bereits der im Rahmen des erfindungsgemäßen Verfahrens als Quenchflüssigkeit verwendeten wässrigen Lösung an basischem Alkalisalz zu.

Die radikalische Polymerisation der erfindungsgemäß erhältlichen wässrigen Alkaliacrylat-Lösung zur Herstellung von Superabsorbern kann im übrigen in an sich bekannter Weise, z.B. wie es in der US-A 4666983, der EP-A 785224, der US-A 4286082 und in der EP-A 785223 beschrieben ist, erfolgen.

D.h., die radikalisch zu polymerisierende wässrige Monomerenzusammensetzung wird in der Regel zu wenigstens 50 % des Gesamtgewichtes der in ihr enthaltenen Monomeren, teil- oder vollständig neutralisierte Acrylsäure enthalten. Somit können in ihr gegebenenfalls bis zu 50 % des Gesamtgewichtes der in ihr enthaltenen Monomeren an mit Acrylsäure copolymerisierbaren Monomeren enthalten sein.

Diese copolymerisierbaren Monomeren können auch mehr als eine ethylenisch ungesättigte Doppelbindung aufweisen, die zueinander auch zu Konjugation befindlich sein können. In der Regel enthält ein copolymerisierbares Monomer nicht mehr als 5 ethylenisch ungesättigte Doppelbindungen. Ferner wird die Menge dieser vernetzend wirkenden Monomeren, bezogen auf die Gesamtmenge an in der wässrigen Monomerzusammensetzung enthaltenen Monomeren, in der Regel nicht mehr als 30 Gew.-% betragen.

Die copolymerisierbaren Monomeren können der erfindungsgemäß erhältlichen wässrigen Alkaliacrylat-Lösung sowohl nachträglich und/oder auch synchron zu ihrer Erzeugung zugesetzt werden. Beipielsweise können sie bereits in der erfindungsgemäß als Quenchflüssigkeit zu verwendenden wässrigen Alkalisalzlösung gelöst enthalten sein.

Die radikalische Polymerisation kann, wie bereits eingangs erwähnt, sowohl als Lösungs- oder als Suspensionspolymerisation ausgeführt werden. In der Regel wird dabei ein Hydrogel erhalten, das in ein Hydrogel-bildendes Pulver überführt und in der Regel abschließend oberflächennachvernetzt wird.

In einer häufigen Ausführungsform führt man die Polymerisation als Lösungspolymerisation unter Ausnutzung des Trommsdorff-Norish-Effektes durch.

Üblicherweise wird die radikalisch zu polymerisierende wässrige Monomerenmischung 10 bis 70 % ihres Gewichtes, häufig 20 bis 60 % ihres Gewichtes an Monomeren enthalten.

Dabei wird die wässrige Monomerenmischung in teil- oder vollneutralisierter Form eingesetzt. In der Regel wird der Neutralisationsgrad aller Säuregruppen-tragenden Monomeren 20 bis 100 mol-%, bezogen auf die molare Gesamtmenge an Säuregruppen, betragen. Oft liegt der vorgenannte Neutralisierungsgrad bei 50 bis 100 mol-% bzw. bei 90 bis 100 mol-%. Falls erforderlich, kann der Neutralisationsgrad nachträglich der Erzeugung einer erfindungsgemäßen wässrigen Lösung durch Zusatz von z.B. einer Mineralsäure oder von (zweckmäßigerweise polymerisationsinhibierter) Acrylsäure eingestellt werden.

In der Regel wird die Polymerisation unter weitgehendem oder vollständigem Ausschluss von Sauerstoff durchgeführt. Üblicherweise arbeitet man dabei unter einer Inertgasatmosphäre. Als Inertgas wird insbesondere Stickstoff eingesetzt. Dabei hat es sich bewährt, das zu polymerisierende wässrige Monomerengemisch vor und/oder während der Polymerisation mit Inertgas zu spülen. Aber insbesondere dann, wenn das wässrige Monomergemisch keinen von der entstehenden Acrylsäure herrührenden Lagerpolymerisationsinhibitor aufweist, kann die Polymerisation auch im Beisein von Sauerstoff, d.h., zweckmäßigerweise unter Luft, durchgeführt werden. Die Polymerisation kann z.B. im Temperaturbereich von 0 °C bis 150 °C oder im Bereich von 0 °C bis 100 °C ausgeführt werden.

Ferner kann die Polymerisation sowohl bei Normaldruck als auch unter erhöhtem oder erniedrigtem Druck erfolgen.

Beispiele für mit Acrylsäure copolymerisierbare monoethylenisch ungesättigte Monomere, die ebenfalls eine Säuregruppe oder die zugehörige Anhydridgruppe tragen, sind z.B. monoethylenisch ungesättigte Mono- und Dicarbonsäuren mit vorzugsweise 4 bis 8 C-Atomen wie Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure; Halbester von monoehtylenisch ungesättigten Dicarbonsäuren mit 4 bis 10 vorzugsweise 4 bis 6 C-Atomen, z.B. von Maleinsäure, wie Maleinsäuremonomethylester; monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxy-propylsulfonsäure, Styrolsulfonsäure, 2-Acrylamido-2-methylpropansulfonsäure, Vinylphosphonsäure und Allylphosphonsäure und die Salze, insbesondere die Natrium-, Kalium- und Ammoniumsalze der genannten Säuren.

Bevorzugte Säuregruppen tragende monoethylenisch ungesättigte Comonomere sind Methacrylsäure, Vinylsulfonsäure, 2-Acrylamido-2-methylpropansulfonsäure oder Mischungen dieser Säuren bzw. ihrer Salze.

Der Anteil an den vorgenannten Comonomeren, bezogen auf die Gesamtmonomerenmenge, kann 0,1 bis 30, oder 0,5 bis 20 Gew.-% betragen.

Zur Optimierung von Eigenschaften der Superabsorber kann es sinnvoll sein, auch monoethylenisch ungesättigte Comonomere einzusetzen, die keine Säuregruppe tragen. Hierzu gehören beispielsweise monoethylenisch ungesättigte Nitrile wie Acrylnitril, Methacrylnitril, die Amide der vorgenannten monoethylenisch ungesättigten Carbonsäuren, z.B. Acrylamid, Methacrylamid, N-Vinylamide wie N-Vinylformamid, N-Vinylacetamid, N-Methylvinylacetamid, N-Vinylpyrrolidon und N-Vinylcaprolactam. Außerdem zählen Vinylester gesättigter C₁-C₄-Carbonsäuren wie Vinylformiat, Vinylacetat und Vinylpropionat, Alkylvinylether mit mindestens 2 C-Atomen in der Alkylgruppe, z.B. Ethylvinylether oder Butylvinylether, Ester monoethylenisch ungesättigter C₃-C₆-Carbonsäuren, z.B. Ester aus einwertigen C₁-C₁₈-Alkoholen und Acrylsäure, Methacrylsäure oder Maleinsäure, Acrylsäure- und Methacrylsäureester von alkoxylierten einwertigen, gesättigten Alkoholen, z.B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die zahlenmittleren Molmassen beispielsweise bis zu 2000 betragen können, dazu.

Weitere geeignete, an Säuregruppen freie, monoethylenisch ungesättigte Comonomere sind Styrol und alkylsubstituierte Styrole wie Ethylstyrol oder tert.-Butylstyrol.

Der Anteil an Säuregruppen freien monoethylenisch ungesättigten Comonomeren wird in den Superabsorberharzen in der Regel 20 Gew.-% nicht überschreiten (der Begriff Säuregruppen bezieht in diesem Zusammenhang die konjugierte Base-Gruppe immer mit ein).

Als vernetzend wirkende mehrfach ethylenisch ungesättigte Comonomere kommen insbesondere solche Monomere in Betracht, die 2, 3, 4 oder 5 ethylenisch ungesättigte Doppelbindungen im Molekül aufweisen, die untereinander auch Konjugation aufweisen können. Diese Monomeren sollen hier als Vernetzermonomere bezeichnet werden. Beispiele geeigneter Vernetzermonomere sind N,N'-Methylenbisacrylamid, Polyethylenglykoldiacrylate und Polyethylenglykoldimethacrylate, die sich jeweils von Polyethylenglykolen eines zahlenmittleren Molekulargewichts von 106 bis 8500, vorzugsweise 400 bis 2000, ableiten, Trimethylolpropantriacrylat, Trimethylolpropantrimethacrylat, Ethylenglykoldiacrylat, Ethylenglykoldimethacrylat, Propylenglykoldiacrylat, Propylenglykoldimethacrylat, Butandioldiacrylat, Butandioldimethacrylat, Hexandioldiacrylat, Hexandioldimethacrylat, Diethylenglykoldiacrylat, Diethylenglykoldimethacrylat, Triethylenglykoldiacrylat, Triethylenglykoldimethacrylat, Dipropylenglykoldiacrylat, Dipropylenglykoldimethacrylat, Tripropylenglykoldiacrylat, Tripropylenglykoldimethacrylat, Allylmethacrylat, Diacrylate und Dimethacrylate von Blockcopolymerisaten aus Ethylenoxid und Propylenoxid, zwei-, drei-, vier- oder fünffach mit Acrylsäure oder Methacrylsäure veresterte mehrwertige Alkohole, wie Glycerin, Trimethylolpropan, Penaterythrit oder Dipentaerythrit, Ester monoethylenisch ungesättigter Carbonsäuren mit ethylenisch ungesättigten Alkoholen wie Allylalkohol, Cyclohexanol und Dicyclopentylalkohol, z.B: Allylacrylat und Allylmethacrylat, weiterhin Triallylamin, Dialkyldiallylammoniumhalogenide wie Demethyldiallylammoniumchlorid und Diethyldiallylammoniumchlorid, Tetraallylethylendiamin, Divinylbenzol, Diallylphthalat, Polyethylenglykoldivinylether von Polyethylenglykolen eines zahlenmittleren Molekulargewichtes von 106 bis 4000, Trimethylolpropandiallylether, Butandioldivinylether, Pentaerythrittriallylether, Umsetzungsprodukte von 1 Mol Ethylenglykoldiglycidylether oder Polyethylenglykoldiglycidylether mit 2 Mol Pentaerythritoltriallylether oder Allylalkohol, und Divinylethylenharnstoff. Üblicherweise enthalten Superabsorber 0,01 bis 5 Gew.-%, häufig 0,2 bis 3 Gew.-% an Vernetzermonomeren copolymerisiert.

Als vernetzend wirkende Verbindungen können auch gesättigte oder ungesättigte polyfunktionelle Verbindungen fungieren, die wenigstens zwei (z.B. 2, 3, 4 oder 5) funktionelle Gruppen aufweisen, die hinsichtlich ihrer Reaktivität gegenüber der Carboxylgruppe der Acrylsäure bzw. ihres Alkalisalzes komplementär sind. Als Vernetzer kommen aber selbstredend auch monoethylenisch ungesättigte Verbindungen in Betracht, die zusätzlich zur ethylenisch ungesättigten Doppelbindung eine weitere gegenüber Carboxylgruppen komplementäre funktionelle Gruppe aufweisen. Beispiele hierfür sind Hydroxyalkylacrylate, Hydroxyalkylmethacrylate sowie Glycidylester der (Meth)acrylsäure. In Betracht kommen als Vernetzer auch Polymere mit einer Vielzahl derartiger komplementärer funktioneller Gruppen. Geeignete komplementäre funktionelle Gruppen sind z.B. Hydroxyl, Amino-, Epoxy- und Aziridingruppen, weiterhin Isocyanat-, Ester- und Amidogruppen sowie Alkoxysilylgruppen. Zu den geeigneten Vernetzern dieses Typs zählen beispielsweise Aminoalkohole, wie Ethanolamin oder Triethanolamin, Di- und Polyole, wie 1,3-Butandiol, 1,4-Butandiol, Ethylenglykol, Diethylenglykol, Triethylenglykol, Tetraethylenglykol, Polyethylenglykol, Glycerin, Polyglycerin, Propylenglykol, Polypropylenglykol, Trimethylolpropan, Pentaerythrit, Polyvinylalkohol, Sorbit, Stärke, Blockcopolymerisate aus Ethylenoxid und Propylenoxid, Polyamine wie Ethylendiamin, Diethylentriamin, Triethylentetramin, Tetraethylenpentamin, Pentaethylenhexamin und Polyethylenimine sowie Polyamine mit zahlenmittleren Molmassen von jeweils bis zu 4.000.000, Ester wie Sorbitanfettsäureester, ethoxylierte Sorbitanfettsäureester, Polyglycidylether wie Ethylenglykoldiglycidylether, Polyethylenglykol-diglycidylether, Glycerindglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydoxymethylbutanol-tris[3-(1-aziridinyl)-propionat], Diamide der Kohlensäure wie 1,6-Hexamethylendiethylenharnstoff und Diphenylmethan-bis-4,4'-N,N'-diethylenharnstoff, Halogenepoxyverbindungen wie Epichlorhydrin und α-Methylepifluorhydrin, Polyisocyanate wie 2,4-Toluylendiisocyanat und Hexamethylendiisocyanat, Alkylencarbonate wie 1,3-Dioxolan-2-on und 4-Methyl-1,3-dioxolan-2-on, weiterhin Bisoxazoline und Oxazolidone,Polyamidoamine sowie deren Umsetzungsprodukte mit Epichlorhydrin, ferner polyquaternäre Amine wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Als Polymerisationsreaktoren kommen die zur Herstellung üblichen Reaktoren, im Falle der Lösungspolymerisation insbesondere Bandreaktoren, Extruder und Kneter, in Betracht. Die Polymerisate können insbesondere auch nach einem kontinuierlichen oder diskontinuierlichen Knetverfahren hergestellt werden.

Als Initiatoren kommen grundsätzlich alle Verbindungen in Betracht, die beim Erwärmen auf Polymerisationstemperatur unter Bildung von Radikalen zerfallen. Die Polymerisation kann auch durch Einwirkung energiereicher Strahlung, z.B. UV-Strahlung, in Gegenwart von Photoinitiatoren ausgelöst werden. Auch eine Initiierung der Polymerisation durch Einwirkung von Elektronenstrahlen auf die polymerisierbare, wässrige Mischung ist möglich.

Geeignete Initiatoren sind beispielsweise Peroxoverbindungen wie organische Peroxide, organische Hydroperoxide, Wasserstoffperoxid, Persulfate, Perborate, Azoverbindungen und die sogenannten Redoxinitiatorsysteme. Bevorzugt werden wasserlösliche Initiatoren. In manchen Fällen ist es vorteilhaft, Mischungen verschiedener Polymerisationsinitiatoren zu verwenden, z.B. Mischungen aus Wasserstoffperoxid und Natrium- oder Kaliumperoxodisulfat. Geeignete organische Peroxide sind beispielsweise Acetylacetonperoxid, Methylethylketonperoxid, tert-Butylhydroperoxid, Cumolhydroperoxid, tert-Amylperpivalat, tert-Butylperpivalat, tert-Butylperneohexanoat, tert-Butylperisobutyrat, tert-Butyl-per-2-ethyl-hexanoat, tert-Butylperisononanoat, tert-Butylpermaleat, tert-Butylperbenzoat, Di-(2-ethylhexyl)peroxydicarbonat, Dicyclohexylperoxidicarbonat, Di-(4-tert.-butylcyclohexyl)peroxydicarbonat, Dimyristilperoxidicarhbonat, Diacetylperoxi-dicarbonat, Allylperester, Cumylperoxyneodecanoat, tert.-Butyl-per-3,5,5-trimethylhexanoat, Acetylcyclohexylsulfonylperoxid, Dilaurylperoxid, Dibenzoylperoxid oder tert.-Amylperneodecanoat. Besonders geeignete Polymerisationsinitiatoren sind wasserlösliche Azostarter, z.B. 2,2'-Azo-bis-(2-amidinopropan)dihydrochlorid, 2,2'-Azo-bis-(N,N'-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z.B. in Mengen von 0,01 bis 5, vorzugsweise 0,05 bis 2,0 Gew.-%, bezogen auf die zu polymerisierenden Monomeren.

Bevorzugte Redoxininitiatorsysteme sind wasserlöslich und enthalten als oxidierende Komponente mindestens eine der oben angegebenen Peroxoverbindungen und als reduzierende Komponente beispielsweise Ascorbinsäure, Glukose, Sorbose, Ammonium- oder Alkalimetallsulfit, -hydrogensulfit, -thiosulfat, -hyposulfit, -pyrosulfit oder -sulfid, Metallsalze wie Eisen(II)-ionen oder Natriumhydroxymethylsulfoxylat. Vorzugsweise verwendet man als reduzierende Komponente des Redoxinitiatorsystems Ascorbinsäure oder Natriumsulfit. Bezogen auf die bei der Polymerisation eingesetzte molare Menge an Monomeren verwendet man beispielsweise 3x10⁻⁶ bis 1 Mol-% an reduzierender Komponente des Redoxinitiatorsystems und 0,001 bis 5,0 Mol-% an oxidierender Komponente des Redoxinitiatorsystems.

Wenn man die Polymerisation durch Einwirkung energiereicher Strahlung auslöst, verwendet man üblicherweise als Initiator sogenannte Photoinitiatoren.

Die Herstellung eines Superabsorbers kann auch eine nachträgliche innere Vernetzung des Gels umfassen. Bei der nachträglichen Vernetzung (sog. Gelvernetzung) werden Polymere, die durch die Polymerisation von wenigstens teilneutralisierter Acrylsäure und gegebenenfalls Comonomeren hergestellt wurden, mit Verbindungen umgesetzt, die mindestens zwei gegenüber den Carboxylgruppen reaktive Gruppen aufweisen. Diese Umsetzung kann bei Raumtemperatur oder aber bei erhöhten Temperaturen bis zu 220 °C erfolgen. Zur nachträglichen inneren Vernetzung (Gelvernetzung) werden die Vernetzer den erhaltenen Polymeren in Mengen von 0,5 bis 20 Gew.-%, bevorzugt von 1 bis 14 Gew.-%, bezogen auf die Menge des Polymers, zugesetzt.

Die wie beschrieben erhältlichen Polymerisate fallen in der Regel als Hydrogele an. Ihr Feuchtigkeitsgehalt liegt in der Regel im Bereich 20 bis 80 Gew.-%. Das so erhaltene Hydrogel wird dann in an sich bekannter Weise in ein Hydrogel bildendes Pulver überführt und abschließend in der Regel oberflächennachvernetzt.

Hierzu wird das bei der Polymerisation anfallende Hydrogel in der Regel zunächst nach bekannten Methoden zerkleinert. Die Grobzerkleinerung der Hydrogele erfolgt mittels üblicher Reiß- und/oder Schneidwerkzeuge, z.B. durch die Wirkung einer Austragspumpe im Falle der Polymerisation in einem zylindrischen Reaktor oder durch eine Schneidwalze oder Schneidwalzenkombination im Falle der Bandpolymerisation.

Das so erhaltene voll- oder teilneutralisierte Polymerisat wird anschließend bei erhöhter Temperatur, z.B. im Bereich von 80 °C bis 250 °C und insbesondere im Bereich von 100 °C bis 180 °C, nach bekannten Verfahren getrocknet. Hierbei erhält man die Polymerisate in Form von Pulvern oder Granulaten, die gegebenenfalls zur Einstellung der Partikelgröße noch mehreren Mahl- und Siebvorgängen unterworfen werden.

Die anschließende Oberflächennachvernetzung erfolgt in an sich bekannter Weise mit den so erhaltenen, getrockneten, vorzugsweise gemahlenen und abgesiebten Polymerpartikeln. Zu Oberflächenvernetzung werden Verbindungen eingesetzt, die wenigstens zwei funktionelle Gruppen aufweisen, die mit den funktionellen Gruppen, vorzugsweise den Carboxylgruppen des Polymers unter Vernetzung reagieren können (Nachvernetzungsmittel). Hierzu werden die Nachvernetzungsmittel, vorzugsweise in Form einer wässrigen Lösung, auf die Oberfläche der Polymerisat-Partikel aufgebracht. Die wässrige Lösung kann wassermischbare organische Lösungsmittel enthalten. Geeignete Lösungsmittel sind z.B. C₁-C₄-Alkohole wie Methanol, Ethanol, Isopropanol oder Ketone wie Aceton und Methylethylketon.

Geeignete Nachvernetzungsmittel sind beispielsweise:
- Di- oder Polyglycidylverbindungen wie Phosphonsäurediglycidylether oder Ethylenglykoldiglycidylether, Bischlorhydrinether von Polyalkylenglykolen,
- Alkoxysilylverbindungen,
- Polyaziridine, Aziridin-Einheiten enthaltende Verbindungen auf Basis von Polyethern oder substituierten Kohlenwasserstoffen, beispielsweise Bis-N-aziridinomethan,
- Polyamine oder Polyamidoamine sowie deren Umsetzungsprodukte mit Epichlorhydrin,
- Diole und Polyole, z.B. Ethylenglycol, 1,2-Propandiol, 1,4-Butandiol, Glycerin, Methyltriglykol, Trimethylolethan, Trimethylolpropan, Polyethylenglykole mit einem mittleren Molekulargewicht M_{W} von 200 bis 10000, Di- und Polyglycerin, Pentaerythrit, Sorbit, die Oxethylate dieser Polyole sowie deren Ester mit Carbonsäuren oder mit Kohlensäure wie Ethylencarbonat oder Propylencarbonat,
- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,
- Di- und Poly-N-methylolverbindungen wie beispielsweise Methylenbis(N-methylol-methacrylamid) oder Melamin-Formaldehyd-Harze, und
- Verbindungen mit zwei oder mehr blockierten Isocyanat-Gruppen wie beispielsweise Trimethylhexamethylendiisocyanat blockiert mit 2,2,3,6-Tetramethyl-piperidinon-4.

Bei Bedarf können saure Katalysatoren wie p-Toluolsulfonsäure, Phosphorsäure, Borsäure oder Ammoniumdihydrogenphosphat zugesetzt werden.

Das Aufbringern der Vernetzer-Lösung erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt erfolgen. Dies erfolgt bevorzugt in einem nachgeschalteten Trockner und bei einer Temperatur zwischen 80 und 230 °C, besonders bevorzugt 80 bis 190 °C, und ganz besonders bevorzugt zwischen 100 und 160 °C und über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können. Die Trocknung kann aber auch im Mischer selbst erfolgen. Beispielsweise durch Beheizen des Mantels oder Einblasen eines vorgewärmten Trägergases.

Bevor die erfindungsgemäß erhältliche wässrige Alkaliacrylat-Lösung zu Polymerisationszwecken verwendet wird, kann sie auch noch mit einem mit Wasser begrenzt oder nicht mischbaren organischen Lösungsmittel extrahiert werden. Auf diese Weise können in der wässrigen Alkaliacrylat-Lösung noch enthaltene störende Verunreinigungen, z.B. niedermolekulare Aldehyde aller Art, abgetrennt werden.

Unter einem mit Wasser begrenzt mischbaren organischen Lösungsmittel versteht man dabei insbesondere solche organischen Lösungsmittel, deren Löslichkeit in Wasser bei 20 °C und 1 atm weniger als 10 Gew.-%, vorzugsweise weniger als 7 Gew-% und besonders bevorzugt weniger als 5 Gew.-% beträgt.

Beispiele für derartige Lösungsmittel sind aliphatische und cycloaliphatische Kohlenwasserstoffe wie Hexan, Heptan, Oktan, deren Isomere und Cyclohexan, aromatische Kohlenwasserstoffe wie Toluol, xylole, Ethylbenzol und Cumol, technische Kohlenwasserstoffgemische wie Petrolether, Benzinfraktionen und ähnliche, Ester von aliphatischen C₁-C₄-Carbonsäuren mit C₁-C₆-Alkanolen oder mit Cycloalkanolen und vorzugsweise insgesamt 4 bis 10, insbesondere 5 bis 8 C-Atomen wie n-Butyl- und Isobutylformiat, Ethylacetat, n-Propylacetat, Isopropylacetat, n-Butylacetat, Isobutylacetat, Amylacetat, Isoamylacetat, n-Hexylacetat, Cyclohexylacetat, Methyl-, Ethyl-, n-Propyl-, Isopropyl-, n-Butyl- und Isobutylpropionat, Methyl-, Ethyl-, n-Propyl-, Isopropyln-Butyl- und Isobutylbutyrat und -isobutyrat, aliphatische und cycloaliphatische Ketone mit wenigstens 5 C-Atomen wie Methylpropylketon, Methylisopropylketon, Methylbutylketon, Methylisobutylketon, Methylamylketon, Methylisoamylketon, Diethylketon, Ethylpropylketon, Ethylbutylketon, Diisopropylketon, Diisobutylketon, Cyclohexanon und Trimethylcyclohexanon, aliphatische und cycloaliphatische Ether mit wenigstens 4 C-Atomen wie Diethylether, Methyl-tert.-Butylether, Diisopropylether, Di-n-butylether, Ethyl-tert.-butylether, chlorierte Kohlenwasserstoffe wie Dichlormethan, Chlorethan, Dichlorethan, Trichlorethan, sowie Mischungen der vorgenannten Lösungsmittel.

Bevorzugt sind solche Lösungsmittel, deren Siedepunkt unterhalb 150 °C und insbesondere unter 140 °C bei Normaldruck (1 atm) liegt. Bevorzugt sind insbesondere solche Lösungsmittel, deren Lösungsmittelpolarität durch einen E_{T}(30)-Wert nach C. Reichardt et al. (Liebigs Annalen der Chemie, 1983, S. 721-743) im Bereich von 32 kcal/mol bis 42 kcal/mol und insbesondere im Bereich von 33 bis 40 kcal/mol aufweisen.

Besonders bevorzugte Lösungsmittel sind Methyl-tert.-butylether, Toluol, Ethylacetat, Isobutylacetat und Methylisobutylketon.

Die Extraktion der wässrigen Alkaliacrylat-Lösung kann sowohl kontinuierlich als auch diskontinuierlich erfolgen. Zu Extraktion können z.B. Extraktionsapparaturen verwendet werden, wie sie aus dem Stand der Technik bekannt sind (siehe z.B. Ullmann's Enzyklopädie der Techn. Chem. 4. Aufl., Bd. 2, S. 560ff, Verlag-Chemie Weinheim; Ullmann's Enzyclopedia of Ind. Chem. 5^{th} ed. on CD-Rom, "Liquid-liquid exrtaction 3.1", Wiley-VCH Weinheim 1997; Vauck/Müller, "Grundoperationen chemischer Verfahrenstechnik", 10. Aufl., Deutscher Verlag für Grundstoffindustrie Leipzig, Stuttgart, 1994, S. 777-796). Beispiele für geeignete Solvent-Extraktoren sind Rührextraktoren, Kreiselpumpenextraktoren, Strahlpumpenextraktoren, Ultraschallextraktoren (die vorzugsweise als Extraktionsbatterien angeordnet sind) Mixer-Settler-Apparaturen, Extraktionskolonnen mit ruhenden Einbauten (wie z.B. Füllkörperkolonnen, Siebbodenkolonnen sowie Kaskadenbodenkolonnen) und/oder mit Mitteln zur Pulsation oder mit bewegten Einbauten (z.B. Rührkolonnen, Drehscheibenkolonnen und Quirlkolonnen) sowie Zentrifugalextraktoren.

Die für das Extraktionsverfahren eingesetzten Extraktoren werden vorzugsweise so ausgelegt, daß die theoretische Stufenzahl wenigstens 3, vorzugsweise wenigstens 5 beträgt. Üblicherweise beträgt diese Stufenzahl nicht mehr als 15. Häufigst liegt diese Stufenzahl im Bereich von 5 bis 10.

Gegebenenfalls kann man im Anschluss an den Extraktionsschritt nach Trennung von Extrakt und wässrigem Raffinat den Neutralisationsgrad auf den für den jeweiligen Vezwendungszweck der wässrigen Alkaliacrylat-Lösung erforderlichen Wert neu einstellen. Beispielsweise kann man den Neutralisationsgrad durch Zugabe einer Mineralsäure oder einer Acrylsäure verringern oder durch Zugabe von Base wie Alkalihydroxid, Alkalicarbonat oder -hydrogencarbonat erhöhen.

Das bei der Extraktion anfallende organische Lösungsmittel (Extrakt) kann in einfacher Weise destillativ aufgearbeitet werden. Auf diese Weise gewinnt man das Lösungsmittel zurück und kann es in die Extraktion zurückführen.

Das erfindungsgemäße Verfahren ist insbesondere auf Acrylsäure enthaltende Gemische anwendbar, die, bezogen auf ihr Gesamtgewicht, wenigstens 70 Gew.-% an Acrylsäure enthalten. D.h., das erfindungsgemäße Verfahren ist dann anwendbar, wenn der vorstehend beschriebene Gehalt an Acrylsäure wenigstens 80 Gew.-%, oder wenigstens 90 Gew.-%, oder wenigstens 95 Gew.-%, oder wenigstens 97 gew.-% oder wenigstens 99 gew.-% beträgt.

Demnach ist das erfindungsgemäße Verfahren insbesondere anwendbar auf die Acrylsäure enthaltenden Gemische, die in der EP-A 717029, der EP-A 839790, der US-A 5482597, der EP-A 713854, der DE-A 19634614, der US-A 5710329, der EP-A 1 110940, der DE-A 19853064, der DE-A 4201697, der DE-A 3641996, der EP-A 1033359, der DE-A 10138150, der DE-A 10138101 und der EP-A 648732 zum Zweck der Abtrennung von Acrylsäure destillativ behandelt werden.

Selbstredend ist das erfindungsgemäß zu behandelnde Acrylsäure enthaltende Gemisch im Rahmen der erfindungsgemäßen destillativen Behandlung mit Prozeßpolymerisationsinhibitor versetzt. Als Prozeßinhibitoren kommen dabei all jene in Betracht, die zum Stand der Technik, u.a. auch in den vorgenannten Schriften, für Destillationszwecke empfohlen werden. Besonders geeignet ist Phenothiazin als Polymerisationsinhibitor. Bezogen auf die enthaltene Acrylsäuremenge beträgt die Inhibitormenge in der Regel etwa 200 bis 400 Gew.ppm. Zum Zweck der zusätzlichen Polymerisationsinhibierung wird im Rahmen einer Ausübung des erfindungsgemäßen Verfahrens zusätzlich häufig Luft oder mit Stickstoff verdünnte Luft durch die Destillationsvorrichtung geführt und der Rücklauf mit Inhibitcr versetzt.

Für die Durchführung des erfindungsgemäßen Destillationsverfahrens können je nach Beschaffenheit des zu destillierenden Acrylsäure enthaltenden Gemisches die an sich bekannten Destillationsvorrichtungen eingesetzt werden. Diese können Einbauten enthalten oder an Einbauten frei sein. Als Einbauten kommen Packungen, Böden (z.B. Dual-Flow-Böden), Füllkörper (z.B. Raschig-Ringe) oder alle weiteren möglichen Einbauten in Betracht. Wird eine Einbauten aufweisende Destillationsvorrichtung, z.B. eine Kolonne, verwendet, wird man in der Regel noch unterhalb des Entnahmebodens Prozeßpolymerisationsinhibitor in die Destillationsvorrichtung führen. Dieser kann z.B. in einer Acrylsäure von angemessener Reinheit gelöst zugeführt werden.

Die Anwendung des erfindungsgemäßen Verfahrens empfiehlt sich insbesondere für den letzten Destillationsschritt in den nachfolgenden Ketten zur Herstellung von Acrylsäure.

Zunächst werden Propen, Propan und/oder Acrolein in an sich bekannter Weise einer heterogenen katalysierten Gasphasenoxidation zu Acrylsäure unterworfen. Die Durchführung derselben kann z.B. so erfolgen, wie es in den Schriften DE-A 19636489, WO-0196271, DE-A 10028582, DE-A 10122027,DE-A 10121592, DE-A 10119933, DE-A 10118814, DE-A 10101695, DE-A 10063162, DE-A 10051419, DE-A 10033121, DE-A 10028582, DE-A 19955176, DE-A 19955168, DE-A 19948523 und der DE-A 19948248 beschrieben ist.

Aus dem resultierenden Produktgasgemisch wird Acrylsäure entweder durch Absorption in eine Absorptionsflüssigkeit oder durch fraktionierende Kondensation des Produktgasgemisches abgetrennt.

Als Absorptionsflüssigkeiten sind grundsätzlich all jene geeignet, in denen Acrylsäure eine signifikante Löslichkeit aufweist. Bevorzugt werden unter diesen jene, von denen die Acrylsäure durch Destillation mit möglichst geringen Aufwand wieder getrennt werden kann. Solche Lösungsmittel sind z.B. Wasser oder organische Flüssigkeiten, deren Siedetemperatur bei Normaldruck (1 atm) oberhalb der Siedetemperatur der Acrylsäure liegt. Günstige hochsiedende organische Flüssigkeiten weisen bei 1 atm einen Siedepunkt von ≥ 160 °C auf. Beispiele für geeignete hochsiedende organische Flüssigkeiten sind Diphenyl, Diphenylether, Dimethylphthalat, Ethylhexansäure, N-Methylpyrrolidon, Paraffinfraktionen oder Gemische davon. Günstig ist insbesondere die Verwendung von hydrophoben organischen Flüssigkeiten wie sie die DE-A 2136396, die DE-A 4436243 und die DE-A 4308087 empfehlen. Dazu gehören insbesondere hochsiedende organische Flüssigkeiten die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden. Die im folgenden gemachten Ausführungen sind zwar allgemein gültig, beziehen sich aber insbesondere auf eine Verwendung eines Gemisches aus 70 bis 75 Gew.-% Diphenylether und 25 bis 30 Gew.-% Diphenyl sowie auf eine Verwendung einer Mischung aus 70 bis 75 Gew.-%Diphenylether und 25 bis 30 Gew.-% Diphenyl, und, bezogen auf diese Mischung, 0,1 bis 25 Gew.-% o-Dimethylphthalat.

Zweckmäßigerweise wird die Absorption im Gegenstrom durchgeführt. Ferner ist günstig, wenn das Produktgasgemisch der Gasphasenoxidation vorab der Absorption abgekühlt wird. Dies kann durch direkte und/oder indirekte Kühlung erfolgen. Eine direkte Kühlung kann beispielsweise wie in der DE-A 10063161, oder wie in der DE-A 2449780 oder wie in der DE-A 4308087 beschrieben durch Teilverdampfen einer hochsiedenden Kühlflüssigkeit (vorzugsweise wird dazu die spätere Absorptionsflüssigkeit verwendet) in einem Direktkühler (Quenchappparat) verwirklicht werden.

Die nachfolgende Absorption selbst kann z.B. so wie in der DE-A 10115277, der EP-A 1125912, der DE-B 2136396, der DE-A 19838817, der DE-A 4436243 und der DE-A 4308087 beschrieben durchgeführt werden.

Aus dem resultierenden Absorbat kann anschließend wie in der DE-A 19838783, der DE-A 19838795, der DE-A 19838817, der DE-A 10115277 und der DE-A 19606877 beschrieben auf rektifikativem Weg eine Roh-Acrylsäure abgetrennt werden.

Ein alternatives Verfahren zur Abtrennung einer rohen Acrylsäure aus dem Produktgasgemisch einer heterogen katalysierten Gasphasenoxidation bietet die fraktionierende Kondensation des Produktgasgemisches, wie es in der DE-A 10217121, der DE-A 10053086, der DE-A 19924533 und der DE-A 19909929 beschrieben ist.

Die so erhaltene rohe Acrylsäure kann bei noch ausgeprägtem Verunreinigungsgehalt zum Zweck der Weiterreinigung zunächst noch einer Kristallisation zugeführt werden. Das dabei anzuwendende, in der Regel nur eine Reinigungsstufe umfassende, Kristallisationsverfahren unterliegt keiner Beschränkung. D.h., es kann sowohl eine Schichtkristallisation, (z.B. eine dynamische Schichtkristallisation wie z.B. die Fallfilmkristallisation oder eine statische Schichtkristallisation an eingetauchten gekühlten Flächen) als auch eine Suspensionskristallisation sein. Die dabei anfallende Acrylsäure soll hier ebenfalls noch als rohe Acrylsäure bezeichnet werden.

Die wie vorstehend beschrieben erhältlichen rohen Acrylsäuren weisen in der Regel noch einen für die in dieser Schrift beschriebenen Acrylsäurefolgeverwendungen nicht tolerablen Gehalt an aldehydischen Verunreinigungen auf.

In zweckmäßiger Weise wird man eine solche Roh-Acrylsäure daher zunächst mit einem Aldehydfänger behandeln. Die Zugabe des Aldehydfängers kann direkt in eine Rohrleitung, mittels welcher die Rohacrylsäure der weitern Aufarbeitung zugeführt wird, oder in einen Verweilzeitbehälter hinein erfolgen, in welchem die Roh-Acrylsäure zwischengelagert wird, bevor man sie der weiteren Aufarbeitung zuführt.

Als Aldehydfänger sind alle Verbindungen geeignet, die die in der Roh-Acrylsäure enthaltenen Aldehyde im wesentlichen quantitativ in Verbindungen mit einem höheren Siedepunkt als Acrylsäure umwandeln. Hierzu eignen sich besonders Stickstoffverbindungen mit wenigstens einer primären Aminogruppe (vgl. EP-A 648732, EP-A 717029, EP-A 713854, US-A 5,482,597, EP-A 1 110940 und DE-A 3641996). Als Beispiele lassen sich Aminoguanidinsalze, Hydrazin, Alkyl- und Arylhydrazine, Carbonsäurehydrazide und Aminophenole aufführen. Aminoguanidinhydrogencarbonat ist unter ihnen besonders bevorzugt.

Der Aldehydfänger wird vorzugsweise im Überschuss zu dem in der Roh-Acrylsäure enthaltenen Aldehyd, z.B. in einer Menge von 1,5 bis 2,5 Mol Aldehyd, eingesetzt. Zur Reaktion mit dem Aldehydfänger ist eine Temperatur von 15 bis 50 °C, vorzugsweise 20 bis 30 °C, ausreichend. Üblicherweise hält man Reaktionsdauern von 10 Minuten bis 72 Stunden, vorzugsweise 2 bis 50 Stunden, ein. Wird Aminoguanidinhydrogencarbonat als Aldehydfänger eingesetzt, so bildet sich unter Kohlendioxidentwicklung zunächst Aminoguanidinhydrogenacrylat. Dieses reagiert mit den Aldehydgruppen der vorhandenen Aldehyde zu den entsprechenden Iminoguanidin-Derivaten bzw. deren Umlagerungsprodukten. Durch die Behandlung mit dem Aldehydfänger läßt sich der Restaldehydgehalt der Roh-Acrylsäure an freiem Aldehyd, ausgedrückt als Furfural, auf unter 20 Gew. ppm, insbesondere auf unter 5 Gew.ppm und ganz besonders auf unter 3 Gew.ppm senken.

Die wie vorstehend behandelte Roh-Acrylsäure kann nun auf unterschiedliche Art und Weise erfindungsgemäß in eine wässrige Alkaliacrylat-Lösung überführt werden.

Im einfachsten Fall wird die behandelte Roh-Acrylsäure an einer Destillationsvorrichtung thermisch in Acrylsäure-haltige Brüden und einen hochsiedenden Rückstand aufgetrennt. Die thermische Trennung kann durch einfache Destillation, d.h., im wesentlichen ohne Rücklauf von Kondensat, oder rektifikativ erfolgen. Im ersten Fall verwendet man zweckmäßigerweise eine Destillationskolonne ohne trennwirksame Einbauten, d.h., ein hohles säulen- oder turmähnliches Gebilde, das in der Regel aus Edelstahl gefertigt ist. Um zu verhindern, daß von den Acrylsäure-haltigen Brüden Tröpfchen von Roh-Acrylsäure mitgerissen werden, ist die Kolonne zweckmäßigerweise mit einem Tropfenabscheider üblicher Bauart, z.B. in Form einer Drahtgestrick-Packung mit großer innerer Oberfläche, die z.B. aus Chromnickelstählen, Aluminium, Polypropylen, Polytetrafluorethylen oder dergleichen gefertigt sein kann, oder in Form einer Füllkörperschüttung oder gerichteten Packung, z.B. eines Stapels zueinander beabstandeter, parallel zur Längsachse der Kolonne angeordneter gewellter Bleche geringer Höhe von z.B. 20 bis 100 cm, ausgerüstet.

Die Sumpftemperatur liegt üblicherweise bei etwa 65 bis 130 °C, vorzugsweise 70 bis 100 °C, der Kolonnendruck bei 50 bis 120 mbar. Die Sumpfheizung der Kolonne erfolgt durch einen außen oder innen liegenden Umlaufverdampfer, vorzugsweise einen Robert-Verdampfer oder einen Zwangsumlaufentspannungsverdampfer. Bei Verdampfern des Robert-Typs ist in einem zylindrischen Verdampferkörper ein Heizkörper mit senkrechten Siederohren untergebracht. Die Rohacrylatsäure befindet sich im Inneren der Siederohre. Die Zirkulation in den Rohren wird durch die aufsteigenden Dampfblasen bewirkt. Zur Rückführung der nach oben geförderten Flüssigkeit sind im Heizkörper ein oder mehrere Fallrohre angebracht.

Abgesehen von der beschriebenen Sumpfheizung wird die Kolonne vorzugsweise nicht aktiv beheizt; der Kolonnenmantel ist jedoch vorzugsweise isoliert, um einen übermäßigen Wärmeverlust durch Wärmestrahlung zu vermeiden. Der Verzicht auf eine Kolonnenheizung (abgesehen von der Sumpfheizung) bewirkt, daß Tröpfchen von Rohacrylsäure, die von Acrylsäure-haltigen Brüden mitgerissen werden, auf ihrem Weg durch den Gasraum der Kolonne nicht erwärmt werden und sich ihre Größe durch Abdampfen flüchtiger Bestandteile nicht verringert. Die mitgerissenen Tröpfchen, die ihre Größe beibehalten oder durch Koagulation vergrößern, können dann bei der Passage der Acrylsäure-haltigen Brüden durch einen Tropfenabscheider gut zurückgehalten werden.

Abgesehen vom Kolonnenmantel sind die übrigen Teile der Anlage, die mit den Acrylsäure-haltigen Brüden in Kontakt kommen, insbesondere die Rohrleitungen, in denen die Acrylsäure-haltigen Brüden bis zu ihrer Kondensation geführt werden, mit einer Begleitheizung versehen, um eine unerwünschte vorzeitige Kondensation zu vermeiden. So können die Rohrleitungen z.B. als Doppelmantelrohr ausgeführt sein, in dessen Ringraum zwischen äußerem und innerem Mantel ein Heizmedium zirkuliert wird. Alternativ kann man ein von einem Heizmedium durchströmtes Rohr vorsehen, das mit der Rohrleitung, das die Acrylsäure-haltigen Brüden führt, in wärmeleitendem Kontakt steht, und z.B. spiralig um dieses gewicklt ist oder parallel dazu verläuft.

Die Destillation wird in der Regel so geführt, daß der Rückstand wenigstens 8 Gew.-%, z.B. 8 bis 30 Gew.-%, vorzugsweise 10 bis 25 Gew.-%, der der Destillationskolonne zugeführten behandelten Roh-Acrylsäure ausmacht. Auf diese Weise erreicht man, daß der Rückstand eine handhabbare, nicht zu hohe Viskosität aufweist. Außerdem wurde gefunden, daß es beim weitergehenden Eindampfen zu einer signifikant stärkeren Belagbildung ("Fouling") auf den Wärmetauschflächen der zur Sumpfheizung der Kolonne herangezogenen Verdampfer kommt, die in kurzen Zeitabständen zum Abstellen und Reinigen der Anlage zwingt.

Zur Gewinnung der im ersten Rückstand noch enthaltenen Acrylsäure führt man diesen einem Filmverdampfer zu und erhält eine weitere Menge Acrylsäure-haltiger Brüden. Als Filmverdampfer sind Wischblattverdampfer besonders geeignet. Bei diesem Typ wird die einzudampfende Flüssigkeit durch eine rotierende Anordnung von Wischblättern über eine Röhrenwand verteilt. Verdampfer vom Sambay-Typ sind besonders bevorzugt. Es wurde gefunden, daß Filmverdampfer aufgrund ihrer Bauart eine geringe Neigung zur Belagsbildung zeigen und so ein stärkeres Eindampfen des Rückstands ohne Reinigungsunterbrechung erlauben, als dies in der primären Destillationskolonne möglich ist. Der erste Rückstand wird im Filmverdampfer vorzugsweise auf 35 % bis 5 %, insbesondere 10 % bis 20 %, aufkonzentriert.

Die zweite Menge Brüden kann man mit der ersten Menge Brüden vereinigt werden, geeigneterweise, indem man die zweite Menge Brüden in die Destillationskolonne zurückführt. Zweckmäßigerweise führt man die zweite Menge Brüden unterhalb eines in der Destillationskolonne vorgesehenen Tropfenabscheiders ein und führt die vereinigten Brüden durch den Tropfenabscheider. Diese Verfahrensführung weist den Vorteil auf, daß zur Abtrennung mitgerissener Tröpfchen aus der ersten und der zweiten Menge Brüden nur ein gemeinsamer Tropfenabscheider erforderlich ist, was die Investitionskosten und den Reinigungsaufwand senkt. Die am Filmverdampfer anfallenden Rückstände, die z.B. 0,5 bis 5 Gew.-%, in der Regel 1 bis 2 Gew.-%, des gesamten Roh-Acrylsäure-Zulaufs entsprechen, werden verworfen.

In einer bevorzugten Ausführungsform erwärmt man die mit dem Aldehydfänger behandelte Roh-Acrylsäure vor dem Einführen in die Destillationskolonne auf eine Temperatur von 40 bis 110 °C, vorzugsweise 50 bis 60 °C. Das Erwärmen erfolgt geeigneterweise durch indirekten Wärmetausch, z.B. mittels eines Durchlaufwärmetauschers. Das Einführen vorerwärmter Roh-Acrylsäure in die Destillationskolonne hat den Vorteil, daß im Kolonnensumpf eine geringere Wärmemenge über den dafür vorgesehenen Verdampfer aufgebracht werden muss, was wiederum zu einer verringerten Belagbildung an dessen Wärmetauschflächen führt.

Bei der thermischen Trennung der Roh-Acrylsäure wird zweckmäßigerweise ein Spaltungskatalysator für oligomere Acrylsäure, insbesondere Diacrylsäure, mitverwendet, insbesondere Säuren wie Alkyl- und Arylsulfonsäuren, wie z.B. Dodecylbenzolsulfonsäure oder p-Toluolsulfonsäure, oder Basen wie Natriumhydroxid oder Kaliumcarbonat. Der Spaltungskatalysator wird üblicherweise in einer Menge von 0,5 bis 10 kg pro Tonne Roh-Acrylsäure verwendet. Die Zugabe des Spaltungskatalysators kann zum Roh-Acrylsäurezulauf oder zum Zulauf der Filmverdampfers erfolgen.

Die Acrylsäure-haltigen Brüden werden erfindungsgemäß behandelt, d.h., unmittelbar aus der Gasphase in eine wässrige Lösung eines Alkalihydroxids, eines Alkalicarbonats und/oder eines Alkalihydrogencarbonats aufgenommen, wobei sich die gewünschte polymerisationsfähige wässrige Alkaliacrylat-Lösung bildet.

Wird die Abtrennung der Acrylsäure rektifikativ durchgeführt, kann ein- oder zweistufig vorgegangen werden. Wird einstufig vorgegangen, besteht der wesentliche Unterschied zum eben Beschriebenen darin, daß die Kolonne Einbauten (z.B. Füllkörper, Böden und/oder Packungen) enthalten.

Wird zweistufig vorgegangen, dies ist insbesondere dann empfehlenswert, wenn die behandelte Roh-Acrylsäure noch signifikante Anteile an leichtsiedenden nicht aldehydischen Nebenkomponenten wie Essigsäure, Ameisensäure etc. enthält, können von der behandelten Roh-Acrylsäure in einer ersten Retifikatiionsstufe diese leichter siedenden Komponenten in Begleitung von Acrylsäure über Kopf abgetrennt (und konventionell kondensiert werden) und diese Acrylsäurefraktion für Zwecke der Herstellung von Acrylsäureestern (z.B. Methylacrylat, Ethylacrylat, Butylacrylat, 2-Ethylhexylacrylat etc.) weiterverwendet werden. Die Sumpfflüssigkeit würde dann wie oben beschrieben destillativ und/oder rektifikativ weiterbehandelt und die Acrylsäure-haltigen Brüden erfindungsgemäß unmittelbar aus der Gasphase in eine wässrige Alkalilösung aufgenommen werden.

Das erfindungsgemäße Verfahren kann aber auch auf die in der DE-A 10218419 beschriebenen Verfahren zur rektifikativen Abtrennung von (Meth)acrylsäure angewendet werden.

Die wie vorstehend beschrieben erhältlichen wässrigen Alkaliacrylat-Lösungen eignen sich in hervorragender Weise zur unmittelbaren Herstellung wasserabsorbierender Harze auf Polyacrylatbasis.

Selbstredend werden die beschriebenen Destillations- und/oder Rektifikationsverfahren im Beisein von Prozeßinhibitoren wie Phenothiazin durchgeführt.Sie sind üblicherweise sowohl im Zulauf zu den Kolonnen, als auch im Rücklauf (soweit vorhanden) enthälten.

Zum Zweck der zusätzlichen Polymerisationsinhibierung führt man häufig noch Sauerstoff -enthaltende Gase, z.B. Luft, durch die Kolonnen.

Zur Verhinderung von Fouling werden die beschriebenen Rektifikationen vielfach zusätzlich im Beisein von organischen Sulfonsäuren wie Dodecylbenzolsulfonsäure und/oder im Beisein von Tensiden durchgeführt, wie es z.B. die EP-A 648732, die EP-A 713854, die US-A 5482597, die EP-A 839790, die DE-A 19810962 und die DE-A 4335172 beschreiben und empfehlen.

### Beispiel

In eine 45 Dual-Flow-Böden (Durchmesser der Bohrungen = 15 mm) enthaltende Rektifikationskolonne 1 werden auf den 40. Boden je Stunde 14 m³ einer mit Aminoguanidinhydrogencarbonat behandelten Roh-Acrylsäure und 60 kg Dodecylbenzolsulfonsäure geführt.

Die Zusammensetzung der unbehandelten Roh-Acrylsäure umfasst dabei:
98 Gew.-% Acrylsäure,
250 Gew. ppm Phenothiazin,
300 Gew. ppm Furfurale,
50 Gew. ppm Benzaldehyd,
50 Gew. ppm Allylacrylat,
150 Gew. ppm Essigsäure,
350 Gew. ppm Propionsäure,
1500 Gew. ppm Wasser.

Die dieser Roh-Acrylsäure zugeführte Menge Aminoguanidinhydrogencarbonat (AGHC) ist so bemessen, daß sie je Mol der in der Roh-Acrylsäure als Nebenkomponenten enthaltenen Aldehyde 2,0 Mol beträgt.

Die Rektifikationskolonne 1 wird bei einem Sumpfdruck von 130 mbar und einer Sumpftemperatur von 86 °C betrieben. Zur Stabilisierung der Kolonne werden durch den Sumpf der Kolonne 2,5 Nm³/h Luft geführt.

Über ihren Rücklauf (3 m³/h) wird die Kolonne polymerisationsinhibiert.

Dazu wird ein Teil (200 l/h) der Kopfabnahme (Leichtsieder enthaltende Acrylsäure) mit 1 % ihres Gewichtes an Phenothiazin versetzt und in den Rücklauf gegeben. Mit einem weiteren Teil (150 l/h) an 1 % ihres Gewichtes Phenothiazin zugesetzt enthaltender Kopfabnahme wird die nicht in die Kolonne rückgeführte Kopfabnahme (1,4 m³/h) stabilisiert. Sie kann zur Herstellung von Alkylestern der Acrylsäure verwendet werden.

Die Kondensation am Kopf der Kolonne erfolgt durch Direktkühlung (Quenchkreis) mittels gekühlter (auf eine Temperatur von 30 °C) zuvor abgetrennter und inhibierter Kopfabnahme. Der dabei verbleibende Abgasstrom wird mittels eines nachgeschalteten zweiten mit Wasser (18 °C) betriebenen, Quenchkreises von Acrylsäure frei gewaschen.

Der jeweilige Quenchraum wird nach unten jeweils durch einen Kaminboden abgeschlossen. Das Waschwasser kann in einer Kläranlage entsorgt werden. Der Begriff Quenchkreis bringt zum Ausdruck, daß ein Teil des gebildeten Kondensats über einen Wärmetauscher geführt, gekühlt und zur Verstärkung der Kühlwirkung als zusätzliche Quenchflüssigkeit im Kreis in die Kolonne rückgeführt wird.

Die Direktkühlung kann auch in einem Quenchraum außerhalb der Rektifikationskolonne 1 erfolgen.

Diesem wird das Gasgemisch von oben zugeführt und mit der versprühten gekühlten Quenchflüssigkeit im Gleichstrom von oben nach unten geführt (am unteren Ende erfolgt die Kondensationsentnahme). Kurz vor dem unteren Ende befindet sich ein Seitenarm, über den das Abgas den Quench verlässt. In diesem Seitenarm wird das Abgas nochmals nachgewaschen, indem es dem Gegenstrom der Sprühtropfen eines zweiten Quench ausgesetzt wird. Das Temperaturniveau des zweiten Quench liegt unterhalb des Temperaturniveau des ersten Quench. Als Quenchflüssigkeit wird jeweils gekühlte zuvor abgetrennte und inhibierte Kopfabnahme verwendet.

Der Sumpf der Rrektifikationskolonne 1 wird in einer Menge von 12 m³/h einer Destillationskolonne ohne Einbauten zugeführt. Die Sumpftemperatur derselben beträgt 72 °C bei einem Sumpfdruck von 78 mbar.

Über Kopf wird hinter einem Tropfenabscheider in einer Menge von 11,7 m³/h gereinigte Acrylsäure (≥ 99,5 Gew.-%) in einem Quenchkreis in 25 gew.-%ige gekühlte wässrige NaOH-Lösung aufgenommen. Kondensations- und Neutralisationswärme werden über parallel geschaltete Plattenwärmetauscher von insgesamt 600 m² Fläche abgeführt. Die Quenchkreistemperatur wird bei 35 bis 40 °C gehalten. Die Natronlaugedosierung wird über eine Verhältnisregelung so eingestellt, daß 75 % der Acrylsäure neutralisiert werden. Der Sumpf der Reinkolonne wird in einem nachgeschalteten Fallfilmverdampfer (75 °C, 70 mbar) bis auf 300 l/h eingeengt und die Brüden unterhalb des Tropfenabscheiders in die Destillationskolonne rückgeführt. Der verbleibende Rückstand wird durch Verbrennen entsorgt.

Die dem Quenchkreis entnommene wässrige Natriumacrylatlösung hat einen Gehalt an Acrylsäure und Natriumacrylat von insgesamt 40 Gew.-%. Eine Stabilisierung des Quenchkreises mit 50 Gew.ppm Monoethylethers des Hydrochion erweist sich als ausreichend.

In einem Laborkneter werden 6000 g der erhaltenen wässrigen Natriumacrylatlösung, 27 g Polyethylenglykol 400-diacrylat, 16,7 g Natriumperoxodisulfat und 0,36 g Ascorbinsäure vorgelegt. Durch die Vorlage wird Stickstoff geleitet. Die Manteltemperatur des Laborkneters wird auf 74 °C angehoben. Durch einsetzende Polymerisation, in deren Verlauf die Temperatur bis auf 88 °C ansteigt, entsteht ein festes Gel, das anschließend bei 160 °C getrocknet, mechanisch zerkleinert und ausgesiebt wird. Ausgesiebt wird ein Polymerisatpulver einer Korngröße von 100 bis 800 µm.

Zur Bestimmung der freien Quellbarkeit werden 0,2 g des getrockneten Polymerisatpulvers in einen 60x85 mm großen Teebeutel eingewogen, der anschließend verschweißt wird. Der Teebeutel wird für 30 Minuten in einen Überschuss von 0,9 gew.-%iger Kochsalzlösung gegeben (wenigstens 0,83 1 Kochsalzlösung/g Polymer). Anschließend wird der Teebeutel 3 Minuten lang bei 250 g zentrifugiert. Die Flüssigkeitsaufnahme beträgt 37,4 g/g Polymer.

Zur Bestimmung der extrahierbaren Anteile werden in einem Becherglas 0,9 g des getrockneten Polymerisatpulvers in 187 ml 0,9 gew.-%iger wässriger Kochsalzlösung suspendiert. Danach wird durch einen 0,22 µm-Filter filtriert und der Gehalt an extrahierbarem durch Säure-Base Titration bestimmt.
Er beträgt 21,8 Gew.-%.

## Patentansprüche

1. Verfahren zur Herstellung einer wässrigen Alkaliacrylat-Lösung, bei dem man aus einem Acrylsäure enthaltenden Gemisch Acrylsäure destillativ abtrennt und aus der destillativ abgetrennten Acrylsäure und einer wässrigen Lösung eines basischen Alkalisalzes eine wässrige Alkaliacrylat-Lösung erzeugt, **dadurch gekennzeichnet, daß** man
a) das die Acrylsäure enthaltende Gemisch einer Destillationsvorrichtung zuführt,
b) die Abtrennung der Acrylsäure vom Acrylsäure enthaltenden Gemisch in der Destillationsvorrichtung oberhalb der Zuführstelle durchführt und
c) die Erzeugung der wässrigen Alkaliacrylat-Lösung so vornimmt, daß die in der Destillationsvorrichtung abgetrennte Acrylsäure unmittelbar aus der Gasphase in eine wässrige Lösung eines Alkalihydroxids, eines Alkalicarbonats und/oder eines Alkalihydrogencarbonats aufgenommen wird.

2. Verfahren nach Anspruch 1, **dadurch gekennzeichnet, dass** sich als Verfahrensschritt d) die Herstellung eines Polyacrylats unter Einpolymerisation der wässrigen Alkaliacrylat-Lösung anschließt.

3. Verfahren nach Anspruch 1, **dadurch gekennzeichnet, dass** die unmittelbare Aufnahme der in der Destillationsvorrichtung abgetrennten Acrylsäure aus der Gasphase in eine wässrige Lösung eines Alkalihydroxids, eines Alkalicarbonats und/oder eines Alkalihydrogencarbonats in einer Polymerisationsvorrichtung erfolgt.

## Revendications

1. Procédé de préparation d'une solution aqueuse d'acrylate de métal alcalin, dans lequel on isole de l'acide acrylique par distillation à partir d'un mélange contenant de l'acide acrylique et on produit, à partir de l'acide acrylique isolé par distillation et d'une solution aqueuse d'un sel de métal alcalin basique, une solution aqueuse d'acrylate de métal alcalin, **caractérisé en ce que**
a) on alimente un dispositif de distillation en le mélange contenant l'acide acrylique,
b) on effectue l'isolement de l'acide acrylique à partir du mélange contenant de l'acide acrylique dans le dispositif de distillation, au-dessus de l'emplacement d'alimentation, et
c) on effectue la production de la solution aqueuse d'acrylate de métal alcalin de façon que l'acide acrylique isolé dans le dispositif de distillation soit immédiatement absorbé depuis la phase gazeuse dans une solution aqueuse d'un hydroxyde de métal alcalin, d'un carbonate de métal alcalin et/ou d'un bicarbonate de métal alcalin.

2. Procédé suivant la revendication 1, **caractérisé en ce que**, comme étape d) du procédé, fait suite la préparation d'un polyacrylate avec copolymérisation de la solution aqueuse d'acrylate de métal alcalin.

3. Procédé suivant la revendication 1, **caractérisé en ce que** l'absorption immédiate de l'acide acrylique isolé dans le dispositif de distillation à partir de la phase gazeuse dans une solution aqueuse d'un hydroxyde de métal alcalin, d'un carbonate de métal alcalin et/ou d'un bicarbonate de métal alcalin a lieu dans un dispositif de polymérisation.

## Claims

1. A process for preparing an aqueous alkali metal acrylate solution by distillatively removing acrylic acid from an acrylic acid-comprising mixture and generating an aqueous alkali metal acrylate solution from the distillatively removed acrylic acid and an aqueous solution of a basic alkali metal salt, which comprises
a) feeding the acrylic acid-comprising mixture to a distillation apparatus,
b) carrying out the removal of the acrylic acid from the acrylic acid-comprising mixture in the distillation apparatus above the feed point and
c) generating the aqueous alkali metal acrylate solution in such a manner that the acrylic acid removed in the distillation apparatus is taken up immediately from the gas phase into an aqueous solution of an alkali metal hydroxide, an alkali metal carbonate and/or an alkali metal hydrogencarbonate.

2. The process according to claim 1, which is followed by, as process step d), the preparation of a polyacrylate by polymerizing the aqueous alkali acrylate solution.

3. The process according to claim 1, wherein the acrylic acid removed in the distillation apparatus is taken up immediately from the gas phase into an aqueous solution of an alkali metal hydroxide, an alkali metal carbonate and/or an alkali metal hydrogencarbonate in a polymerization apparatus.
